(19) Europäisches Patentamt / European Patent Office / Office européen des brevets

(11) **EP 3 334 759 B1**

(12) **EUROPEAN PATENT SPECIFICATION**

(45) Date of publication and mention
of the grant of the patent:
**04.09.2019 Bulletin 2019/36**

(51) Int Cl.:
***C07K 14/79*** (2006.01)          ***A61K 38/40*** (2006.01)
***A61K 38/00*** (2006.01)

(21) Application number: **16763573.9**

(22) Date of filing: **28.07.2016**

(86) International application number:
**PCT/IB2016/054513**

(87) International publication number:
**WO 2017/025846 (16.02.2017 Gazette 2017/07)**

(54) **LACTOFERRIN FRAGMENT FOR THE USE AS ANTIBACTERIAL AND ANTIVIRAL AGENT**

LACTOFERRIN-FRAGMENT ZUR VERWENDUNG ALS BAKTERIZID UND VIRUZID

FRAGMENT DE LACTOFERRINE DESTINÉ À ÊTRE UTILISÉ EN TANT QU'AGENT ANTIBACTÉRIEN ET ANTIVIRAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.08.2015 IT UB20153058**

(43) Date of publication of application:
**20.06.2018 Bulletin 2018/25**

(73) Proprietor: **Farmagens Health Care Srl**
**00191 Roma (IT)**

(72) Inventors:
• **SANNETTI, Augusto**
**I-00168 Roma (IT)**
• **PAGANO, Francesco**
**I-81030 Cancello ed Arnone (CE) (IT)**
• **GALDIERO, Stefania**
**I-80123 Napoli (IT)**
• **GALDIERO, Massimiliano**
**I-80123 Napoli (IT)**

(74) Representative: **Sarpi, Maurizio et al**
**Studio Ferrario S.r.l.**
**Via Collina, 36**
**00187 Roma (IT)**

(56) References cited:
**WO-A1-2004/089986      WO-A2-00/12542**

• **RASTOGI NILISHA ET AL: "Preparation and Antimicrobial Action of Three Tryptic Digested Functional Molecules of Bovine Lactoferrin", PLOS ONE, vol. 9, no. 3, March 2014 (2014-03), XP002756217,**
• **Marieke I.A Van Der Kraan ET AL: "Lactoferrampin: a novel antimicrobial peptide in the N1-domain of bovine lactoferrin", Peptides, 1 January 2004 (2004-01-01), pages 177-183, XP055263437, United States DOI: 10.1016/j.peptides.2003.12.006 Retrieved from the Internet: URL:http://www.sciencedirect.com/science/article/pii/S0196978103004121/pdfft?md5=23b a10c3fd4822f2b9ad62723ace4f48&pid=1-s2.0-S 0196978103004121-main.pdf [retrieved on 2016-04-06]**
• **GHOSH ANIRBAN ET AL: "Sequence context induced antimicrobial activity: insight into lipopolysaccharide permeabilization", MOLECULAR BIOSYSTEMS, vol. 10, no. 6, 2014, pages 1596-1612, XP002756218,**
• **VAN DER KRAAN M I A ET AL: "Ultrastructural effects of antimicrobial peptides from bovine lactoferrin on the membranes of Candida albicans and Escherichia coli", PEPTIDES, ELSEVIER, AMSTERDAM, NL, vol. 26, no. 9, 1 September 2005 (2005-09-01), pages 1537-1542, XP027856455, ISSN: 0196-9781 [retrieved on 2005-09-01]**

SEQUENCE LISTING

<110> FARMAGENS HEALTHCARE SRL

<120> LACTOFERRIN FRAGMENT FOR THE USE AS ANTIBACTERIAL AND ANTIVIRAL AGENT

<130> 9536

<150> IT102015000044047
<151> 2015-08-11

<160> 2

<170> BiSSAP 1.3

<210> 1
<211> 26
<212> PRT
<213> Bos taurus

<400> 1

```
    Lys Phe Gly Lys Asn Lys Ser Arg Ser Phe Gln Leu Phe Gly Ser Pro
    1               5                   10                  15
    Pro Gly Gln Arg Asp Leu Leu Phe Lys Asp
                20                  25
```

<210> 2
<211> 30
<212> PRT
<213> Bos taurus

<400> 2

```
    Tyr Thr Ala Gly Lys Cys Gly Leu Val Pro Val Leu Ala Glu Asn Arg
    1               5                   10                  15
    Lys Thr Ser Lys Tyr Ser Ser Leu Asp Cys Val Leu Arg Pro
                20                  25                  30
```

**Description**

Technical field

The present invention relates to a novel fragment of lactoferrin for use as an antimicrobial and antiviral agent. The invention relates to an antimicrobial and antiviral agent consisting of at least a bovine lactoferrin peptide fragment as active ingredient.

Background of the invention

Lactoferrin is an 80 kDa glycoprotein binding the iron present in most fluids excreted from the body, including tears, bile, bronchial mucus, gastrointestinal fluids, cervicovaginal mucus, seminal fluid and milk. Lactoferrin is the main constituent of secondary specific granules of circulating polymorphonuclear neutrophils. Source of the most abundant lactoferrin is mammalian milk and colostrum.

Lactoferrin circulates in the body at the concentration of 2-7 $\mu$g/ml, and carries out many biological functions, including the regulation of iron metabolism, immune function, and embryonic development; it is well documented its antimicrobial function against a broad range of pathogens, including Gram positive and Gram-negative bacteria, yeasts and fungi. The antimicrobial effect is partially due to its ability in binding iron, which is essential for the growth of pathogens. Lactoferrin also inhibits replication of several viruses and increases susceptibility of some bacteria to antibiotics and lysozyme through the binding of the lipid A component of lipopolysaccharides on bacterial membranes.

However, lactoferrin antibacterial activity is not very powerful, therefore, in order to exploit its activity, a considerable amount of protein is required. That is undoubtedly representing a limitation to its utility as antimicrobial agent.

An attempt to increase the antibacterial activity of lactoferrin was made; for example, in Japanese patent application No. 62 (1987) 249931 the combined use of lactoferrin with lysozyme has been proposed. It was also reported that the association of lactoferrin and IgA can increase the antibacterial activity of the protein.

Another problem related to the use of lactoferrin in general, and to its use as an antibacterial agent in particular, is the heat instability, in fact, the lactoferrin antibacterial activity can go largely lost by heating at 62.5 °C for 30 minutes, while complete deactivation is achieved at a temperature of 70 °C for 15 minutes (Ford J.E. et al., Journal of Pediatrics, 1997, 90:29); therefore, the heat treatment cannot be applied to the substrates using lactoferrin as antimicrobial agent.

Lactoferrin is also sensitive to variations in pH.

The European patent EP0438750 teaches that lactoferrin hydrolyzate, or apolattoferrin, have an extremely higher antibacterial activity and excellent heat stability than the corresponding non-hydrolized molecule.

An antimicrobial agent containing peptide fragments bearing specific sequence derived from lactoferrin is disclosed in the U.S. patent application US 5,304,633. Also WO2004089986 and WO012542 disclose several lactoferrin peptides having bactericidal or cytotoxic activity. Rastogi et al., 2014 (PloS one, 9(3), page e90011) disclose the identification of three tryptic digested peptides from bovine lactoferrin having antimicrobial activity.

Despite these results, the identification of other forms, or active portions of lactoferrin, able to selectively maintain the antimicrobial and antiviral effect at a high and stable level, is strongly felt in the field of research and development of antimicrobial peptides for possible therapeutic applications.

Summary of the invention

A first object of the present invention is to provide a new peptide fragment of bovine lactoferrin, and its derivatives, having a specific amino acid sequence and antimicrobial and antiviral activity.

A second object of the present invention is to provide an antimicrobial and antiviral agent comprising said peptide fragment of bovine lactoferrin, and its derivatives, as active principle.

A third object of the present invention is a pharmaceutical composition comprising said peptide fragment of bovine lactoferrin, and its derivatives, and/or said antimicrobial agent comprising said peptide of lactoferrin, and its derivatives, alone or in combination thereof, as the active ingredient.

Finally, a last object of the present invention is the use as antimicrobial and antiviral agent of the peptide fragment of bovine lactoferrin, and its derivatives, the use of the active agent comprising said bovine lactoferrin peptide, and its derivatives, and the use of the compositions comprising them.

Description of the figures

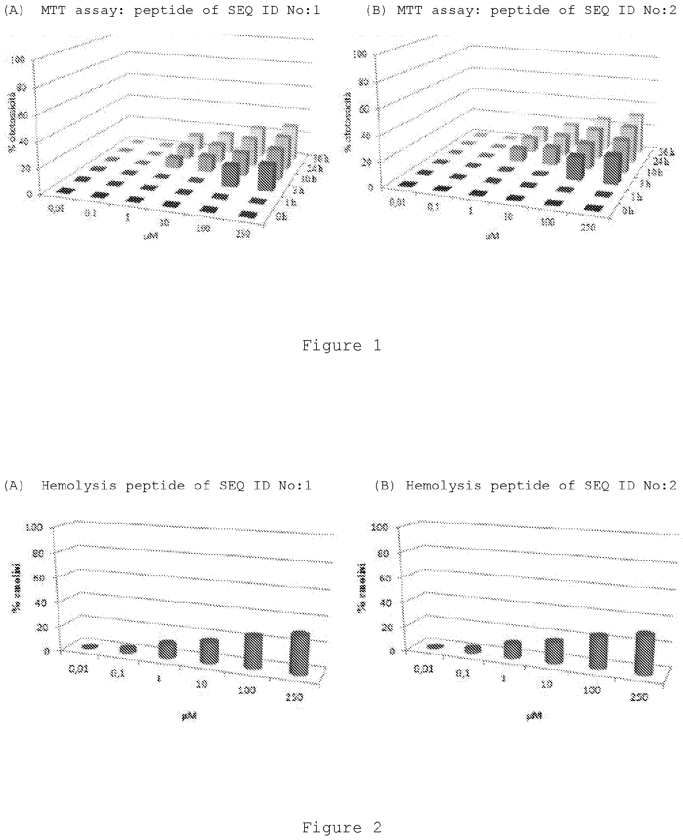

Figure 1 shows the graph of the *in vitro* toxicity test. Figure 1 (A) shows the MTT assay of peptide of SEQ ID No.

1; Figure 1 (B) shows the MTT assay of peptide of SEQ ID No. 2 which is not part of the invention.

Figure 2 shows the graph of the hemolysis assay.

Figure 2 (A) shows the percentage hemolysis induced by the peptide of SEQ ID No. 1 at various concentrations; Figure 2 (B) shows the percentage hemolysis induced by the peptide of SEQ ID No. 2, which is not part of the invention, at various concentrations.

Figure 3 shows the graph of the antibacterial activity at various concentrations of the peptides of SEQ ID No. 1, and SEQ ID No. 2 which is not part of the invention.

Figure 3 (A) shows the graph of the percentage inhibition against the *E. coli*. Figure 3 (B) shows the graph of the percentage inhibition against *S. aureus*.

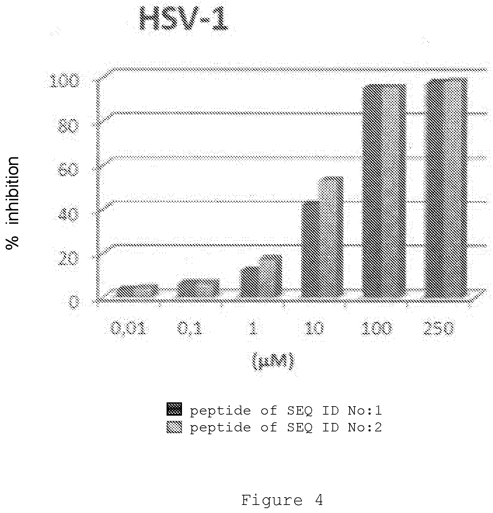

Figure 4 shows the graph of the antiviral activity at various concentrations of the peptides of SEQ ID No. 1, and SEQ ID No. 2 which is not part of the invention.

Detailed description of the invention

The present invention describes a peptide derived from bovine (*Bos taurus*) lactoferrin used as broad-spectrum inhibitors against infections caused by a variety of pathogens. These pathogens may include Gram-positive or Gram-negative bacteria, fungal pathogens, and DNA or RNA viruses. The novel lactoferrin peptide according to the present invention have shown antifungal, antibacterial and antiviral activity, adequate stability, and lack of toxicity *in vitro* in mammalian cells.

The increasing number of infections caused by multi-resistant bacteria to antibiotics is currently a major threat to human health. There is now an urgent need for new drugs, preferably with different mechanisms of action to avert the problem of cross-resistance, as well as new strategies for fighting emerging multidrug-resistant Gram-negative pathogens.

Various bioactive peptides have been described both in scientific and patents literature. Generally, bioactive peptides are isolated from natural sources, and have recently been subject-matter of in-depth structure-function analysis studies. Also, natural peptides are used as starting points for designing synthetic peptide analogs. Bioactive peptides are typically cationic amphipathic molecules of 12 to 45 amino acids in length, permeabilize cell membranes and exert antimicrobial activity. The antibacterial, antifungal, antiviral, anticancer, and wound healing features of bioactive peptides have been extensively described. Structural parameters deemed able to modulate activity and selectivity thereof include helicity, hydrophobic moment, hydrophobicity, and charge. A number of natural and synthetic peptides is already largely present in the literature, however, there exists the need for improved peptides and methods for their use.

Not to be overlooked the fact that using therapeutic peptides is however difficult because of the need to maintain, despite the limited length, the peptide specific three-dimensional structure allowing to maintain the biological activity.

Furthermore, the production and biosynthetic procedures often fail, or have a low yield, precisely due to the lack of, or limited, stability of the peptide during the biosynthesis and purification procedures.

Bovine lactoferrin is a 689 amino acids multifunctional globular protein naturally present in different biological fluids, such as milk, saliva, tears. The protein approximately weighs 80 kDa and includes two homologous regions. Lactoferrin has significant antioxidant, antitumor and immunomodulatory features including immunotropic and anti-inflammatory properties. The typical antimicrobial activity of lactoferrin is due to the strong affinity of the two homologous regions for $Fe^{3+}$ ions which are subtracted from the surrounding environment, thereby preventing the proliferation of microorganisms. Furthermore, the combination of lactoferrin with the ferric ion in the mucous secretions modulates the activity and the aggregating ability of bacteria and viruses to the cell membranes. The iron-independent bactericidal activity is able to attack and lyse the bacterial membrane by exploiting the affinity of its cationic domains to the membrane which is negatively charged. Specifically, the protein is positively charged, directly interacts with the surface of the bacterial cell, particularly with the lipopolysaccharide (LPS) of Gram negative bacteria and with the tecoic and lipotecoico acid of Gram-positive bacteria. Such interactions with bacterial membranes were observed by electron microscopy techniques. *In vivo* the partial degradation of lactoferrin by gastric pepsin releases peptides, which, as documented by numerous studies, have not only antimicrobial, but also antiviral and antifungal, activity.

The present invention relates to the use of a peptide derived from bovine lactoferrin for the prevention and inhibition of microorganisms proliferation. In particular, the invention provides a method for preventing the microorganisms infection by administration of an effective amount of the pharmaceutical formulation comprising the peptide derived from lactoferrin. In particular, a peptide derived from bovine lactoferrin having antimicrobial, antifungal and antiviral activity is described.

The herein described antimicrobial peptide provides a significant advantage compared to currently used antimicrobial agents. The use of antimicrobial peptides provides, in fact, a valid alternative to the current drugs for the treatment of viral and bacterial infections. The peptides in general are versatile in that, for example, they can be designed for interrupting the life cycle of a micro-organism with mechanisms of action different from those of nucleoside analogues, that may be used as antiviral agents and antibiotics or acting in a metabolic stage where the resistance is less likely to develop. The peptides also provide the advantage that they can be synthesized in large amount at relatively low cost and can be easily modified to express different properties. The choice of particular amino acids, or amino acid analogues, incorporated into a peptide will depend, in part, by physical, chemical or biological specific applications of the antimicrobial peptide.

In the present description the terms "peptide", "peptide fragment" and "polypeptide" are synonymously used, and unless otherwise specified, refer to polymeric nitrogen organic compounds resulting from the combination of two or more amino acids joined by peptide bonds, arising from the cleavage of proteins. The term also includes oligopeptides, protein fragments, analogues and protein derivatives, pegylated derivatives, glycosylated derivatives, and the like as commonly understood by skilled persons.

In one embodiment of the invention the bovine (*Bos taurus*) lactoferrin peptide comprises a 26 amino acids fragment corresponding to amino acid region extending from residue 296 to residue 321 of the bovine lactoferrin protein (Accession number: B9VPZ5) and having the sequence of SEQ ID NO. 1: KFGKNKSRSFQLFGSPPGQRDLLFKD.

Each amino acid residue in the peptide according to the invention can be in (D)- or (L)- isomeric configuration, to the extent that the peptide retains its functional properties. The choice of including an (L)- or (D)- amino acid in an antimicrobial peptide of the present invention depends, in part, on the desired characteristics of the antimicrobial peptide. For example, the incorporation of one or more (D)- amino acids may confer increased stability to the peptide either *in vitro* or *in vivo*. The inclusion of one or more (D)- amino acids can also increase or decrease the antimicrobial activity of the peptide. Therefore, the inclusion of "D" isomeric forms, compared to the more common "L" ones, can provide particularly useful antimicrobial peptides.

In one embodiment of the invention, the sequence of the peptide according to the invention coincides exactly with the sequence of the corresponding region of the lactoferrin.

Alternatively, in some embodiments, the peptide sequence may also contain amino acid derivatives and non-natural amino acids, that are listed in the group of $\alpha$-amino acids, that are most commonly found in natural proteins, also known as standard amino acids (A. Lehninger Biochemistry, Second Edn. Zanichelli). In fact, among the amino acids other than the natural ones present in peptides according to the invention there are: norleucine, omofenilalanina, ornithine, citrulline, $\beta$-amino acids, $\gamma$-amino acids, $\delta$-amino acids, etc ....

Non-natural amino acids are widely used even to modify the behavior of the proteins, which they are incorporated in, to alter hydrophilicity and hydrophobicity of certain protein domains, improve the functionality of certain enzymes, make less flexible the conformation of proteins, improve pharmacodynamics and bioavailability of certain drugs.

According to the invention the replacement of some natural amino acids with mimetic, modified or non-natural ones can help to assume a greater stability to the final peptide.

Therefore, also homologous peptides containing derivatives or non-natural amino acids, bearing non-protein side chains, are falling within the scope of the present invention. According to the invention homologues and derivatives having a terminal carboxylic acid, or a carboxamide, or a reduced terminal alcohol, or any pharmaceutically acceptable salt, such as a metal salt, e.g. sodium, potassium, lithium or calcium, or a salt with an organic base, or a salt with a mineral acid, including sulfuric acid, hydrochloric acid, or phosphoric acid, or an organic acid, e.g. acetic acid or maleic acid, can be used. Generally, any pharmaceutically acceptable salt can be used provided that the biological properties of the peptide are retained.

The homologues peptides according to the invention may have one or more residues modified by reaction of the side chains or functional groups. Such derivatives include for example, molecules having free amino groups forming amine hydrochlorides, sulfonic groups, carbobenzoxylic groups, t-butiloxycarbonilic groups, acetic chlorine groups, formyl group. The free carboxyl groups may be derivatized to form salts and esters; the free hydroxyl groups may be derivatized to form O-acyl derivatives, or O-alkyl derivatives. Amino acid derivatives comprising phenyl groups, alkylphenyl, trifluoromethylphenyl, pyrazinyl, pyridinyl, thienyl, naphthyl, pirenil are also included.

More specifically, some residues of the peptide according to the invention may not be limited to natural amino acids, such as alpha-L-, or alpha-D-amino acids, or their isomers, but also include non-natural amino acids such as: D-, L-naphtylalanine, D-, L-phenylglycine, D-, L-2-tienylalanine, D-, L-3-tienylalanine, D-, L-1, 2, 3, 4-pireneylalanine, D-, L-2-(pirinidyl)-alanine, D-, or L-3-(pirinidyl)-alanine, D-, L-2-(pyrazinyl)-alanine, D-, L- (isopropyl)-phenylglicine, D-(trifluoromethyl)-phenylglicine, D-(trifluoromethyl)-phenylalanine, D-rho-fluorophenylalanine, D-, L-rho-biphenylphenylalanine, D-, L-rho-metoxybiphenylphenylalanine, D-, L-2-indole (alkyl)alanine, and D-, L -alkylalanine wherein the alkyl group is methyl, ethyl, propyl, hexyl, pentyl, isopropyl, isobutyl, sec-isotil, isopentyl.

The substitution is for all residues equally likely. The choice of changes to be made will be dictated by the analysis of each modification, mainly to obtain homologues with greater antimicrobial activity and stability, but lower toxicity. The amount of residues that can be simultaneously substituted is equal to 20%.

Furthermore, according to the invention a peptide derivative can differ from the natural sequence because of a chemical modification which includes, without limitation, $NH_2$ terminal acylation, acetylation, amidation with mercaptoacetic acids, terminal carboxylamidation with ammonia, methylamine, and the like.

Therefore, within the scope of the invention there are also peptides having at least about 80% sequence identity with the peptide fragment of SEQ ID No..

The peptide according to the invention can be produced by all methods known to the skilled persons in the sector. Peptides may be isolated from lactoferrin hydrolysates for subsequent purification with conventional methods, alternatively, peptides may be conveniently produced by recombinant DNA technologies according to Sambrock et al. 1989, 1992, 2001, Molecular Cloning: A laboratory Manual, Cold Spring Harbour Laboratory, New York.

Preferably, peptide according to the invention are chemically synthesized. Below, in the experimental section, an example of antimicrobial and antiviral peptide synthesis procedure according to the invention is reported.

According to the invention, peptide having antimicrobial and antiviral activity, and their derivatives, can be used as such, without further modification, or in combination with further antibacterial and antiviral agents. Peptides may be administered to various animal species organisms and Humans through food, non-limiting examples are: chewing gum, candy, diet bars, drinks, juices, milk, functional drinks, milk and yoghurt products, capsules, granular powder, tablets. Peptide according to the invention can also be used in medicinal pharmaceutical products and in non-medicinal pharmaceutical products, non-limiting examples are: mouthwashes, antiperspirants, deodorants for the skin, cosmetics, hair lotions and conditioners as creams, toothpaste, products for feminine hygiene, products for hygiene of children, products destined to geriatric age users as adhesive paste for dentures, diapers.

A very broad scope of application of the peptide according to the invention relates to the cleaning and detergents industry, in fact, said peptides, without limitation, can be incorporated in the composition of soaps, detergents, shampoos, personal care products, products for laundry and cleaning of the house, including clothes and paper towels.

Furthermore, the peptide according to the invention can be added or sprayed, or used to coat, impregnate or treat any material or product wherein the prevention and inhibition of microorganisms proliferation is pursued and desired.

In a particularly preferred embodiment of the invention the peptide according to the invention, alone or in combination with further bacterial and antiviral agents, constitute the active principle of pharmaceutical compositions having antimicrobial and antiviral activity comprising said peptides and at least a pharmaceutically acceptable vehicle. Said pharmaceutical compositions may further comprise solvents, stabilizers and excipients, such as aqueous solutions, buffered physiological saline, polyethylene glycol, stearic acid and its magnesium and calcium salts, arabic gum and xanthan, fat emulsions, glycerine, vegetable or injectable organic esters oils. The stabilizing agents include, without limitation, carbohydrates, such as glucose, sucrose, dextrans, starch, maltodextrins, pullulans, silica, talc, antioxidants, such as ascorbic acid, glutathione, chelating agents, low molecular weight proteins and other compounds widely known to those skilled in pharmaceutical field.

The pharmaceutical composition of the present invention is in a form suitable to be administered by oral, intravaginal, rectal, parenteral, intravenous, intramuscular, subcutaneous, intraorbital, intraperitoneal via or by passive or facilitated absorption through the skin. In order to facilitate the transfer of the antibacterial or antiviral treatment to the recipient subject, the pharmaceutical composition may also be incorporated into liposomes, microspheres or other polymeric matrices.

The lactoferrin peptide according to the invention are present in the pharmaceutical composition in a variable amount ranging between 0.01 and 2 milligrams, preferably in an amount between 0.1 and 1 milligram per gram of composition. In particular, the pharmaceutical composition having antibacterial and antiviral activity according to the invention comprises the peptide fragment of SEQ ID No: 1, and its homologues and derivatives exhibiting at least about 80% sequence identity with the peptide fragment of sequence SEQ ID NO. 1, in a total amount ranging between 0.01 and 2 milligrams per gram of composition.

The total effective amount of the peptide according to the invention depends on the individual needs of the subject to be treated; severity of the infection and route of administration can be varied at the discretion of the attending physician.

The peptide fragment according to the invention has demonstrated outstanding antibacterial and antiviral activity, while maintaining the properties of complete lactoferrin, as demonstrated by the experiments presented in the below experimental section of this description.

EXPERIMENTAL SECTION

The invention will be now illustrated with reference to examples and modes of use and experimental assays not limitative of the scope of the invention.

EXAMPLES

Example 1 - Peptide preparation

Peptides were synthesized using standard 9-fluorenylmethoxycarbonyl method on a multispecific PSSM8 peptide synthesizer (Shimadzu Corporation Biotechnology, Kyoto, Japan). The resin NovaSyn TGA (Merck, Darmstadt, Germany) (substitution 0.25 mmol/g) was used as support in solid phase, and synthesis were performed on a scale of 100 micromol. Ten equivalents of the first amino acid were coupled in the resin according to the N,N'-Dicyclohexylcarbodiimide (DIC)/4-Dimethylaminopyridine (DMAP) method (10 equivalents of Fmoc amino acid, 5 equivalents of DIC, 0.1 equivalents of DMAP). All subsequent amino acids, 4 equivalents relative to the resin, were coupled according to the method of N-hydroxybenzotriazole (HOBT)/2-Benzotriazole-3-tetramethyluronium tetrafluoroborate (TBTU)/Di-isopropylethylamine (DIPEA) [1 equivalent of Fmoc amino acid, 1 equivalent of TBTU, one equivalent of HOBT (0.5 mM HOBT in NMP), 2 equivalents of DIPEA (DIPEA 1 mM in N-methylpyrrolidone, NMP)]. The Fmoc protecting group was removed with 30% piperidine in NMP (v/v) . The peptides were fully deprotected and cleaved from the resin by trifluoroacetic acid (TFA) with 5% thioanisole, 3% ethanedithiol, and 2% anisole as scavenger. The crude peptides were precipitated in ice in ethyl ether, filtered, dissolved in water, freeze dried and reduced with dithiothreitol (DTT).

The peptides were purified to homogeneity by chromatography on preparative reverse phase high pressure liquid (RP-HPLC) on chromatographic equipment Schimadzu Class-LC8A, equipped with a Lambda UV detector SPD-10A. Samples were injected on a C18 Phenomenex (column Phenomenex, Torrance, CA, USA) (22 mm x 25 cm, 5 mm), eluted with a mixture of solvents consisting of $H_2O$/0.1% TFA (A) and $CH_3CN$/0.1% TFA (B). A linear gradient from 5 to 50% of B for 17 min at a flow rate of 20 ml/min was used. The collected fractions were freeze-dried and analyzed by reversed phase HPLC on a Shimadzu Class-LCIO equipped with SPD-M10AVP Diode Array Detector using a Phenomenex C18 analytical column (4.6 x 250 mm, 5 mm). The identity of purified peptides was confirmed by liquid chromatography/mass spectrometry (LC-MS ESI) using a Thermo Electron Surveyor MSQ.

Results

The yield of the obtained peptides was between 20 and 30% in the purified product to 99%. As known to the skilled in the field, such yield is very good for the synthesis of peptides of a similar length; furthermore, the synthesis and purification procedure resulted to be extremely reproducible. All homologue peptides containing modified amino acid residues were obtained by solid phase synthesis with similar methodologies to native sequences, appropriate changes to the synthetic strategy, also known to the skilled in the field.

Example 2 - Toxicity assay

MTT assay: cells of Vero cell line (ATCC CRL-1586™ kidney epithelial cells of *Cercopithecus aethiops*) were cultivated at the appropriate growth conditions as recommended by the supplier in 96-well plates at a concentration of $2 \times 10^4$ cells/well and treated with the peptides according to the invention at different concentrations diluted in growth medium (1, 5, 10, 20, 50, 100, 250 mM) for 3, 10 and 24 hours. Subsequently, the medium was aspirated and the cells incubated at 37 °C for 3 hours with 10 μl of MTT (bromide of 3-(4,5-dimethylthiazol-2-yl)-2,5-diphenyltetrazolium), a vital dye able to enter the mitochondria and be metabolized by succinate dehydrogenase complex to formazan salts, determining a violet coloration in those cells.

Afterward, the supernatant was discharged and isopropanol (100 uL) was added to each well to dissolve the produced salts. The reaction was allowed to proceed by incubation at room temperature in tilting agitation for 20 minutes, then spectrophotometric reading was performed at 570nm to assess cell viability in terms of proliferative inhibition and cell growth.

Results

The obtained results, shown in Figure 1, have shown that both peptides according to the invention are not toxic against eukaryotic cells, in fact, only at high concentrations the two peptide variants of the invention determine a maximum 20% toxicity, measured as a function of cellular integrity in the experimental system. Figures 1 (A) and 1 (B) show the results of the MTT assay carried out on Vero cells treated with the peptide of SEQ ID No. 1 (Fig. 1 (A)) and with the peptide of SEQ ID No. 2, which is not part of the invention, (Fig. 1 (B)) at different concentrations. Example 3 - Evaluation of haemolytic activity of the peptides.

Human red blood cells from healthy donors have been isolated and used to performed the hemolytic assay. The peptide fragments of lactoferrin of SEQ ID No. 1 and SEQ ID No. 2 at different concentrations (1, 5, 10, 20, 50, 100, 250 mM) were added to the suspension of erythrocytes to a final volume of 100 μl. Samples were incubated under stirring at 37 °C for 60 min. The hemoglobin release was monitored by measuring the absorbance (Abs) of the supernatant at 540 nm. As negative control, i.e. zero hemolysis (blank), red cells resuspended in PBS were used, whereas completely lysed erythrocytes were used as a positive control for 100% hemolysis. The percentage of hemolysis was calculated using the following equation:

```
%   Hemolysis   =   [(Sample   Abs   -   negative   control
Abs)/(positive control Abs - negative control Abs)] x 100.
```

Results

The haemolytic assay results obtained by using the lactoferrin peptides according to the invention have shown that the two peptides are not toxic towards the erythrocytes. Figures 2 (A) and 2 (B) show the release of hemoglobin monitored by measuring the absorbance (Abs) of the supernatant at 540 nm, with peptide of SEQ ID No. 1 (Fig. 2 (A)), respectively, and peptide of SEQ ID No. 2, which is not part of the invention, (Fig. 2(B)) at different concentrations.

Example 4 - Antibacterial activity

Gram negative *Escherichia coli* (ATCC 11229) and Gram positive *Staphylococcus aureus* (ATCC 6538) were incubated in suitable medium at 37 °C to reach a visible turbidity. The inoculum was then resuspended in 0.9% sterile saline to reach a $OD_{600}$ value corresponding to a concentration of about $1 \times 10^8$ cfu/mL. This standardized inoculum was diluted 1:10 in suitable medium and inoculum concentration was confirmed by colony counts.
The evaluation of antimicrobial activity was performed using the microdilution method in 96-washing buffer. Serial dilutions of the peptide (1, 5, 10, 20, 50, 100, 250 mM) were respectively prepared in PBS to reach a final volume of 100 microliters per well, 10 microliters of standardized inoculum were added to each well (corresponding to a final concentration of about $4 \times 10^5$ cfu/mL). Culture media only was used as negative control and culture medium seeded with the microbial inoculum was used as positive control. Plates were incubated at 37 °C for 24 hours.
Minimum inhibitory concentrations (MIC) was determined by spectrophotometric measurement.

Results

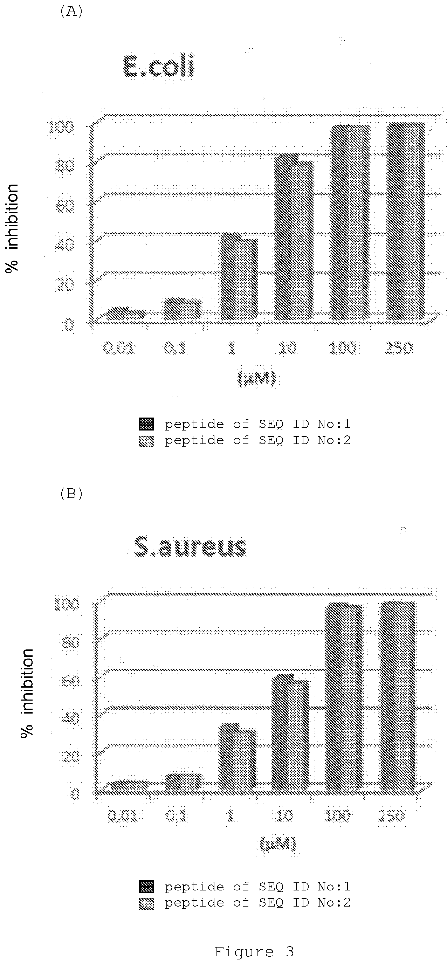

The experimental data obtained by analyzing the antibacterial activity against common pathogens, such as *E. coli* and *S. aureus,* have shown that both lactoferrin peptide fragments according to the invention, SEQ ID No. 1 and SEQ ID No. 2, which is not part of the invention, have a good antimicrobial activity higher than 60% at 10 $\mu$M towards *E. coli,* Figure 3 (A). Peptide of SEQ ID No. 2 activity is reduced, while remaining always higher than 40% at 10 $\mu$M, Figure 3 (B). The peptide of the present invention therefore have excellent antibacterial activity and represent valid compounds which can be used as basic embodiments for further modifications as presented in the description.

Example 5 - Antiviral activity

Cells of the Vero cell line were cultured in Dulbecco's modified Eagle medium (DMEM) supplemented with fetal calf serum to 10%. The HSV-1 (Herpes Simplex Virus Type 1) containing the lacZ gene under the control of the IE-1 promoter of the cytomegalovirus (CMV) expressing beta-galactosidase has been propagated as described in Galdiero M. Site-directed linker and insertion mutagenesis of herpes simplex virus type 1 glycoprotein HJ Virol 1997, 71: 2163-70. The peptides were dissolved in DMEM medium with serum and used at different concentrations. The experiment was carried out in parallel with mixtures of peptides and negative controls. In order to evaluate the effect of the peptides on the inhibition of HSV infectivity, cell monolayers were treated as follows.

i) "co-exposure" experiment: cells were incubated with increasing peptide concentrations (1, 5, 10, 20, 50, 100, 250 mM) and with the viral inoculum for 45 min at 37 °C. The non-penetrated virus were inactivated by the addition of citrate buffer at pH 3.0 after 45 min of incubation with the cells at 37 °C. The cells were then incubated for 48 hours at 37 °C in DMEM supplemented with carboxymethylcellulose (CMC). The monolayers were fixed, stained with 5-bromo-4-chloro-3-indolyl-beta-D-galactopyranoside (X-Gal) and the lysis plaques counted. The experiments were carried out in triplicate and the percentage of inhibition was calculated as compared to control experiments.
ii) "pre-treatment to the virus" experiment: about $2 \times 10^4$ forming units of HSV-1 plaques (UFP) were incubated in the presence of 20 $\mu$M of peptide for 45 min at 37 °C, then titrated on monolayers of Vero cells.
iii) "pre-exposure of the cells" experiment: Vero cells were incubated with 20 $\mu$M of peptide for 30 min at 4 °C. The peptides were removed and the cells were washed with PBS before being infected with serial dilutions of HSV-1 and incubated for 45 min at 37 °C.

Results

The experimental data obtained by analyzing the antiviral activity against viruses with envelope (HSV-1) have shown a good antiviral activity with an IC50 equal to 10μM. Therefore, the peptide of the present invention have excellent antiviral activity and represent a valid compound to be used as basic embodiment for further modifications as presented in the description.

**Claims**

1. Peptide fragment corresponding to the amino acid region extending from amino acid residue 296 to 321 of the bovine lactoferrin protein (Accession number B9VPZ5) and having sequence SEQ ID NO. 1, and its homologues and derivatives with at least about 80% sequence identity with the peptide fragment of sequence SEQ ID NO. 1, wherein the peptide has antibacterial and antiviral activity.

2. Peptide fragment according to claim 1 wherein each amino acid residue can be present as a (D)- or (L)- configuration isomer.

3. Peptide fragment according to claim 1 and 2 wherein the peptide homologue and derivative comprises 20% of non-natural amino acids, β-amino acids, γ-amino acids, δ-amino acids, amino acid derivatives with non-protein side chains.

4. Peptide fragment according to claim 3 wherein the amino acid homologue and derivative has a terminal carboxylic acid, or a carboxamide, or a reduced terminal alcohol, or any pharmaceutically acceptable salt thereof.

5. Peptide fragment according to claim 3 **characterized by** the fact that the amino acid derivative includes molecules wherein the free amino groups can form amine hydrochlorides, sulphonic groups, carbobenzoxy groups, t-butyloxycarbonyl groups, chloro acetic groups, formyl group, wherein the free carboxyl groups can be derivatized to form salts and esters, the free hydroxyl groups may be derivatized to form O-acyl derivatives, or O-alkyl derivatives.

6. Peptide fragment according to claim 3 wherein the amino acid derivative derives from a chemical modification comprising the terminal NH2 acylation, acetylation, amidation with thioglycolic acid, terminal carboxylamidation with ammonia, methylamine, and the like.

7. Peptide fragment according to claim 3 wherein the amino acid derivative includes group: phenyl, alkylphenyl, trifluoromethylphenyl, pyrazinyl, pyridinyl, thienyl, naphthyl, pirenyl.

8. Peptide fragment according to claim 3 wherein the non-natural amino acid is selected from the group of: D-, L-naphthylalanine, D-, L-phenylglycine, D-, L-2-thienylalanine, D-, L-3-thienylalanine, D-, L-1, 2, 3, 4-pirenylalanine, D-, L-2-(pirinidyl)-alanine, D, or L-3-(pirinidyl)-alanine, D-, L-(pyrazinyl)-alanine, D-, L-(isopropyl)-phenylglicine, D-(trifluoromethyl)-phenilglicine, D-(trifluoromethyl) -phenylalanine, D-rho-fluorophenylalanine, D-, L-rho-biphenylphenylalanine, D-, L-rho-metoxybiphenylphenylalanine, D-, L-2-indole(alkyl) alanine, and D-, L-alkylalanine wherein the alkyl group is: methyl, ethyl, propyl, hexyl, pentyl, isopropyl, isobutyl, sec-isotil, isopentyl.

9. Non-therapeutic use of the peptide fragment according to claim 1 as an active ingredient of an antibacterial and antiviral agent.

10. Use according to claim 9 wherein the antibacterial and antiviral agent is used in non-medicinal pharmaceutical products.

11. Use according to claim 10 wherein the non-medicinal pharmaceutical product comprises cleaning products and detergents, soaps, shampoos, detergents, products for the personal hygiene, laundry products and for the cleaning of the house, cloths and absorbent paper, mouthwashes, antiperspirants, deodorants for skin, cosmetics, creams, lotions and hair conditioners, toothpaste, hygiene products for women and children, products intended for elderly users, pasta denture adhesive, absorbent diapers.

12. Peptide fragment corresponding to the region extending from amino acid residue 296 to 321 of the bovine lactoferrin protein and having the sequence SEQ ID NO. 1, and its homologues and derivatives with at least about 80% sequence identity with the peptide fragment of sequence SEQ ID NO. 1 for medical use.

13. Peptide fragment corresponding to the region extending from amino acid residue 296 to 321 of the bovine lactoferrin protein and having the sequence SEQ ID NO. 1, and its homologues and derivatives with at least about 80% sequence identity with the peptide fragment of sequence SEQ ID NO. 1 for use in the prevention and inhibition of bacteria proliferation.

14. A pharmaceutical composition having antibacterial activity and antiviral comprising the peptide fragment of SEQ ID No: 1, and its homologues and derivatives with at least about 80% sequence identity with the peptide fragment of sequence SEQ ID No. 1, alone or in combination with other antibacterial and antiviral agents, and at least one pharmaceutically acceptable vehicle.

15. A pharmaceutical composition having antibacterial activity and antiviral according to claim 14 comprising the peptide fragment of SEQ ID No: 1, and its homologues and derivatives with at least about 80% sequence identity with the peptide fragment of sequence SEQ ID No. 1, in a total amount ranging between 0.01 and 2 milligrams per gram of composition.

16. A pharmaceutical composition having antibacterial and antiviral activity according to claims 14 and 15 further comprising solvents, stabilizers and excipients, aqueous solutions, physiological buffered saline, polyethylene glycol, stearic acid and its magnesium salts and calcium, arabic and xanthan gum, fat emulsions, glycerine, vegetable oils or injectable organic esters, carbohydrates, such as glucose, sucrose, dextrans, starch, maltodextrins, pullulans, silica, talc, antioxidants, ascorbic acid, glutathione, chelating agents, low molecular weight proteins.

17. A pharmaceutical composition having antibacterial and antiviral activity according to claims 14 to 16 in a form suitable to be administered orally, intravaginally, rectally, parenterally, intravenously, intramuscularly, subcutaneously, intraorbitale, intraperitoneal or by passive or facilitated absorption through the skin.

18. A pharmaceutical composition having antibacterial and antiviral activity according to claims 14 to 17 incorporated into liposomes, microspheres or other polymer matrices.

19. A pharmaceutical composition comprising the peptide fragment of SEQ ID No: 1, and its homologues and derivatives with at least about 80% sequence identity with the peptide fragment of sequence SEQ ID No. 1, alone or in combination with other antibacterial and antiviral agents, and at least one pharmaceutically acceptable carrier for use in the prevention and inhibition of microorganisms proliferation.

**Patentansprüche**

1. Peptidfragment, das dem Aminosäurebereich entspricht, der sich vom Aminosäurerest 296 bis 321 des Rinderlaktoferrin-Proteins (Zugangsnummer B9VPZ5) erstreckt, und das die Sequenz SEQ ID NO: 1 aufweist, und deren Homologe und Derivate mit einer Sequenzidentität von mindestens 80% mit dem Peptidfragment der Sequenz von SEQ ID NO: 1, wobei das Peptid eine antibakterielle und antivirale Aktivität aufweist.

2. Peptidfragment nach Anspruch 1, worin jeder Aminosäurerest als (D)- oder (L)- Konfigurationsisomer vorhanden sein kann.

3. Peptidfragment nach Anspruch 1 und 2, wobei das Peptidhomolog und das Peptidderivat 20% an nicht-natürlichen Aminosäuren, β-Aminosäuren, γ-Aminosäuren, δ-Aminosäuren, Aminosäurederivaten mit Nicht-Proteinseitenketten umfasst.

4. Peptidfragment nach Anspruch 3, worin das Aminosäurehomolog und das Aminosäurederivat eine terminale Carbonsäure, oder ein Carboxamid, oder einen reduzierten endständigen Alkohol, oder ein pharmazeutisch akzeptables Salz davon aufweist.

5. Peptidfragment nach Anspruch 3, **gekennzeichnet durch** die Tatsache, dass das Aminosäurederivat Moleküle beinhaltet, worin die freien Aminogruppen Aminhydrochloride, Sulfonsäuregruppen, Carbobenzoxygruppen, t-Butyloxycarbonylgruppen, Chloressigsäuregruppen, eine Formylgruppe, bilden können, worin die freie Carboxylgruppen derivatisiert werden können, um Salze und Ester zu bilden, die freien Hydroxylgruppen derivatisiert werden können, um O-Acyl-Derivate oder O-Alkylderivate zu bilden.

6. Peptidfragment nach Anspruch 3, worin das Aminosäurederivat von einer chemischen Modifikation abstammt, umfassend die terminale NH$_2$-Acylierung, Acetylierung, Amidierung mit Thioglykolsäure, terminale Carboxylamidierung mit Ammoniak, Methylamin, und ähnlichem.

7. Peptidfragment nach Anspruch 3, worin das Aminosäurederivat die nachfolgenden Gruppen beinhaltet: Phenyl, Alkylphenyl, Trifluormethylphenyl, Pyrazinyl, Pyridinyl, Thienyl, Naphthyl, Pirenyl.

8. Peptidfragment nach Anspruch 3, worin die nicht-natürliche Aminosäure ausgewählt ist aus der Gruppe von: D-, L-Naphthylalanin, D-, L-Phenylglycin, D-, L-, 2-Thienylalanin, D-, L-3-Thienylalanin, D-, L-1, 2, 3, 4-Pirenylalanin, D-, L-2-(Pirinidyl)-alanin, D, oder L-3-(Pirinidyl)-alanin, D-, L-(Pyrazinyl)-alanin, D-, L-(Isopropyl)-phenylglycin, D-(Trifluormethyl)-phenylglycin, D-(Trifluormethyl)-phenylalanin, D-rho-Fluorphenylalanin, D-, L-rho-Biphenylphenylalanin, D-, L-rho-Methoxybiphenylphenylphenylalanin, D-, L-2-Indol(alkyl)alanin, und D-, L-Alkylalanin, worin die Alkylgruppe ist: Methyl, Ethyl, Propyl, Hexyl, Pentyl, Isopropyl, Isobutyl, sek.-Isotil, Isopentyl.

9. Nicht-therapeutische Verwendung des Peptidfragments nach Anspruch 1 als Wirkstoff eines antibakteriellen und antiviralen Mittels.

10. Verwendung gemäß nach Anspruch 9, worin das antibakterielle und antivirale in nicht-medizinischen pharmazeutischen Produkten eingesetzt wird.

11. Verwendung nach Anspruch 10, worin das nicht-medizinische Produkt Reinigungsprodukte und Detergentien, Seifen, Shampoos, Waschmittel, Produkte für die Körperpflege, Wäschereiprodukte und Produkte für die Reinigung im Haushalt, von Kleidungsstücken und saugfähigem Papier, Mundwässern, Antitranspirantien, Deodorants für die Behandlung von Haut, Kosmetika, Cremes, Lotionen und Haarspülungen, Zahnpasta, Hygieneprodukte für Frauen und Kinder, Produkte für ältere Menschen, Zahnprothesenhaftmittel, saugfähige Windeln umfasst.

12. Peptidfragment, entsprechend der Region, die sich vom Aminosäurerest 296 bis 321 des Rinderlaktoferrinproteins erstreckt, und mit der Sequenz SEQ ID NO: 1, und seine Homologen und Derivaten mit mindestens etwa 80% Sequenzidentität mit dem Peptidfragment der Sequenz SEQ ID NO: 1, für medizinische Zwecke.

13. Peptidfragment, entsprechend der Region, die sich vom Aminosäurerest 296 bis 321 des Rinderlaktoferrinproteins erstreckt, und mit der Sequenz SEQ ID NO: 1, und seine Homologen und Derivaten mit mindestens etwa 80% Sequenzidentität mit dem Peptidfragment der Sequenz SEQ ID NO: 1 zur Verwendung in der Prävention und Hemmung der Bakterienproliferation.

14. Pharmazeutische Zusammensetzung mit antibakterieller Aktivität und antiviraler Wirkung, die das Peptidfragment von SEQ ID NO: 1 und seine Homologe und Derivate mit mindestens etwa 80% Sequenzidentität mit dem Peptidfragment der Sequenz SEQ ID NO: 1 umfasst, allein oder in Kombination mit anderen antibakteriellen Mitteln und antiviralen Mitteln, und mindestens einem pharmazeutisch akzeptablen Träger.

15. Pharmazeutische Zusammensetzung mit antibakterieller Aktivität und antiviraler Wirkung nach Anspruch 14, umfassend das Peptidfragment von SEQ ID NO: 1, und seine Homologe und Derivate mit mindestens etwa 80% Sequenzidentität mit dem Peptidfragment der Sequenz SEQ ID NO: 1, in einer Gesamtmenge im Bereich zwischen 0,01 und 0,01 und 2 Milligramm pro Gramm der Zusammensetzung.

16. Pharmazeutische Zusammensetzung mit antibakterieller und antiviraler Aktivität gemäß den Ansprüchen 14 und 15, ferner umfassend Lösungsmittel, Stabilisatoren und Hilfsstoffe, wässrige Lösungen, physiologisch gepufferte Kochsalzlösung, Polyethylenglykol, Stearinsäure und ihre Magnesium- und Calciumsalze, Gummi arabicum und Xanthangummi, Fettemulsionen, Glycerin, pflanzliche Öle oder injizierbare organische Ester, Kohlenhydrate, wie Glukose, Sucrose, Dextrane, Stärke, Maltodextrine, Pullulane, Silica, Talkum, Antioxidantien, Ascorbinsäure, Glutathion, Chelatbildner, Wirkstoffe, Proteine mit niedrigem Molekulargewicht.

17. Pharmazeutische Zusammensetzung mit antibakterieller und antiviraler Aktivität gemäß einem der Ansprüche 14 bis 16 in einer Form, die zur Verabreichung in oraler, intravaginaler, rektaler, parenteraler, intravenöser, intramuskulärer, subkutaner, intraorbitaler, intraperitonealer Form geeignet ist, oder durch passive oder erleichterte Absorption durch die Haut.

18. Pharmazeutische Zusammensetzung mit antibakterieller und antiviraler Aktivität gemäß einem der Ansprüche 14 bis 17, die in Liposomen, Mikrokugeln oder in andere Polymermatrices eingebaut ist.

19. Pharmazeutische Zusammensetzung, umfassend das Peptidfragment der SEQ ID NO: 1, und seine Homologe und Derivate mit mindestens etwa 80% Sequenzidentität mit dem Peptidfragment der Sequenz SEQ ID NO: 1, allein oder in Kombination mit anderen antibakteriellen und antiviralen Mitteln, und mindestens einen pharmazeutisch akzeptablen Träger zur Verwendung in der Prävention und Hemmung der Mikroorganismenproliferation.

**Revendications**

1. Fragment peptidique correspondant à la région d'acides aminés s'étendant du résidu d'acide aminé 296 au 321 de la protéine lactoferrine bovine (numéro de dépôt B9VPZ5) et ayant la séquence SEQ ID NO : 1, et ses homologues et dérivés avec une identité de séquence d'au moins environ 80 % avec le fragment peptidique de SEQ ID NO : 1, dans lequel le peptide a une activité antibactérienne et une activité antivirale.

2. Fragment peptidique selon la revendication 1 dans lequel chaque résidu d'acide aminé peut être présent sous la forme d'un isomère de configuration (D) ou (L).

3. Fragment peptidique selon les revendications 1 et 2 dans lequel l'homologue et le dérivé du peptide comprennent 20 % d'acides aminés non naturels, d'acides β-aminés, d'acides γ-aminés, d'acides δ-aminés, de dérivés d'acides aminés avec des chaînes latérales non protéiques.

4. Fragment peptidique selon la revendication 3 dans lequel l'homologue et le dérivé d'acides aminés ont un acide carboxylique terminal, ou un carboxamide, ou un alcool terminal réduit, ou tout sel pharmaceutiquement acceptable de ceux-ci.

5. Fragment peptidique selon la revendication 3 **caractérisé par le fait que** le dérivé d'acides aminés inclut des molécules dans lequel les groupes aminés libres peuvent former des chlorhydrates d'amine, des groupes sulfoniques, des groupes carbobenzoxy, des groupes t-butyloxycarbonyle, des groupes chloro acétiques, un groupe formyle, dans lequel les groupes carboxyle libres peuvent être dérivés pour former des sels et des esters, les groupes hydroxyle libres peuvent être dérivés pour former des dérivés O-acyle, ou des dérivés O-alkyle.

6. Fragment peptidique selon la revendication 3 dans lequel le dérivé d'acides aminés provient d'une modification chimique comprenant l'acylation NH2 terminale, l'acétylation, l'amidation avec l'acide thioglycolique, la carboxylamidation terminale avec l'ammoniac, la méthylamine, et équivalents.

7. Fragment peptidique selon la revendication 3 dans lequel le dérivé d'acides aminés inclut un groupe : phényle, alkylphényle, trifluorométhylphényle, pyrazinyle, pyridinyle, thiényle, naphtyle, pirényle.

8. Fragment peptidique selon la revendication 3 dans lequel l'acide aminé non naturel est sélectionné dans le groupe constitué des : D-, L-naphtylalanine, D-, L-phénylglycine, D-, L- 2-thiénylalanine, D-, L-3-thiénylalanine, D-, L-1, 2, 3, 4-pirénylalanine, D-, L-2-(pirinidyl)-alanine, D, ou L-3- (pirinidyl)-alanine, D-, L-(pyrazinyl)-alanine, D-, L-(isopropyl)-phénylglycine, D-(trifluorométhyl)-phénylglycine, D-(trifluorométhyl)-phénylalanine, D-rho-fluorophénylalanine, D-, L-rho-biphénylphénylalanine, D-, L-rho-méthoxybiphénylphénylalanine, D-, L-2-indole(alkyl)alanine, et D-, L-alkylalanine dans lequel le groupe alkyle est : méthyle, éthyle, propyle, hexyle, pentyle, isopropyle, isobutyle, sec-isotil, isopentyle.

9. Utilisation non thérapeutique du fragment peptidique selon la revendication 1 en tant que principe actif d'un agent antibactérien et antiviral.

10. Utilisation selon la revendication 9 dans laquelle l'agent antibactérien et antiviral est utilisé dans des produits pharmaceutiques non médicaux.

11. Utilisation selon la revendication 10 dans laquelle le produit pharmaceutique non médical comprend des produits de nettoyage et des détergents, des savons, des shampooings, des détergents, des produits pour l'hygiène personnelle, des produits de lessive et des produits pour le nettoyage domestique, des vêtements et des papiers absorbants, des bains de bouche, des anti-transpirants, des déodorants pour la peau, des produits cosmétiques, des crèmes, des lotions et des conditionneurs pour les cheveux, des dentifrices, des produits d'hygiène pour les femmes et les enfants, des produits destinés à des utilisateurs du troisième âge, des adhésifs dentaires en pâte, des couches absorbantes.

12. Fragment peptidique correspondant à la région s'étendant du résidu d'acide aminé 296 au 321 de la protéine lactoferrine bovine et ayant la séquence SEQ ID NO : 1, et ses homologues et dérivés avec une identité de séquence d'au moins environ 80 % avec le fragment peptidique de SEQ ID NO : 1 pour son utilisation médicale.

13. Fragment peptidique correspondant à la région s'étendant du résidu d'acide aminé 296 au 321 de la protéine lactoferrine bovine et ayant la séquence SEQ ID NO : 1, et ses homologues et dérivés avec une identité de séquence d'au moins environ 80 % avec le fragment peptidique de SEQ ID NO : 1 pour son utilisation dans la prévention et l'inhibition de la prolifération bactérienne.

14. Composition pharmaceutique ayant une activité antibactérienne et antivirale comprenant le fragment peptidique de séquence SEQ ID NO : 1, et ses homologues et dérivés avec une identité de séquence d'au moins environ 80 % avec le fragment peptidique de SEQ ID NO : 1, seul ou en combinaison avec d'autres agents antibactériens et antiviraux, et au moins un véhicule pharmaceutiquement acceptable.

15. Composition pharmaceutique ayant une activité antibactérienne et antivirale selon la revendication 14 comprenant le fragment peptidique de SEQ ID NO : 1, et ses homologues et dérivés avec une identité de séquence d'au moins environ 80 % avec le fragment peptidique de SEQ ID NO : 1, en une quantité totale dans la plage de 0,01 à 2 milligrammes par gramme de la composition.

**16.** Composition pharmaceutique ayant une activité antibactérienne et antivirale selon les revendications 14 et 15 comprenant en outre des solvants, des stabilisants et des excipients, des solutions aqueuses, une solution saline tamponnée physiologique, du polyéthylène glycol, de l'acide stéarique et ses sels de magnésium et de calcium, de la gomme arabique et de xanthane, des émulsions grasses, de la glycérine, des huiles végétales ou des esters organiques injectables, des hydrates de carbone, tels que le glucose, le sucrose, des dextranes, de l'amidon, des maltodextines, des pullulanes, de la silice, du talc, des antioxydants, de l'acide ascorbique, du glutathion, des agents chélateurs, des protéines de faible poids moléculaire.

**17.** Composition pharmaceutique ayant une activité antibactérienne et antivirale selon les revendications 14 à 16 sous une forme adaptée à être administrée par voie orale, intravaginale, rectale, parentérale, intraveineuse, intramusculaire, sous-cutanée, intra-orbitale, intrapéritonéale ou par absorption passive ou facilitée à travers la peau.

**18.** Composition pharmaceutique ayant une activité antibactérienne et antivirale selon les revendications 14 à 17 incorporée dans des liposomes, des microsphères ou autres matrices polymères.

**19.** Composition pharmaceutique comprenant le fragment peptidique de SEQ ID NO : 1, et ses homologues et dérivés avec une identité de séquence d'au moins environ 80 % avec le fragment peptidique de séquence SEQ ID NO : 1, seul ou en combinaison avec d'autres agents antibactériens et antiviraux, et au moins un vecteur pharmaceutiquement acceptable pour son utilisation dans la prévention et l'inhibition de la prolifération des microorganismes.

(A) MTT assay: peptide of SEQ ID No:1     (B) MTT assay: peptide of SEQ ID No:2

Figure 1

(A) Hemolysis peptide of SEQ ID No:1     (B) Hemolysis peptide of SEQ ID No:2

Figure 2

(A)

(B)

Figure 3

Figure 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62249931 A
- EP 0438750 A
- US 5304633 A
- WO 2004089986 A
- WO 012542 A
- IT 102015000044047

**Non-patent literature cited in the description**

- **FORD J.E. et al.** *Journal of Pediatrics,* 1997, vol. 90, 29
- **RASTOGI et al.** *PloS one,* 2014, vol. 9 (3), e90011
- **SAMBROCK et al.** Molecular Cloning: A laboratory Manual. Cold Spring Harbour Laboratory, 1989
- **GALDIERO M.** Site-directed linker and insertion mutagenesis of herpes simplex virus type 1 glycoprotein HJ. *Virol,* 1997, vol. 71, 2163-70